# EUROPEAN PATENT APPLICATION

(11) **EP 2 979 672 A1**
(43) Date of publication of application: **03.02.2016**
(21) Application number: 14773034.5
(22) Date of filing: 28.03.2014
(51) Int. Cl.: A61F 13/58, D04H 3/14

(54) **COMPOSITE STRUCTURE FOR ADHESIVE TAPE FOR DIAPER**

(30) Priority: 28.03.2013 CN 201320148926 U; 30.07.2013 CN 201320461413 U
(71) Applicant: Chen, Zhengping, Jiangmen, Guangdong 529000 (CN)
(72) Inventor: Chen, Zhengping, Jiangmen, Guangdong 529000 (CN)
(74) Representative: Ferreccio, Rinaldo
(86) International application number: PCT/CN2014/074284
(87) International publication number: WO 2014/154170

(57) **Abstract**

The present application discloses a fore-attached composite structure for diaper comprising a substrate layer composited by a polymer film, the surface of the substrate layer being composited with a fiber layer of a thermoplastic polymer and having a number of projections, wherein the fiber layer composited on the projections is fluffy; and the fiber layer formed at the recess between each pair of projections is in a dense status. Since the substrate layer composited by the polymer film has the characters of light, soft, and permeable, the fiber layer of a thermoplastic polymer has a soft hand-feel and can be firmly connected the hook&loop.

## Description

### FIELD OF THE INVENTION

The present invention relates to a field of diaper, particularly to an improved fore-attached composite structure for diaper.

### BACKGROUND OF THE INVENTION

In modem society, traditional diaper is gradually being replaced with disposable diaper. Disposable diaper is popular with more and more new parents in recent years due to its advantages of portability, free-washing, easy-replacement and clean, etc.

Conventional diapers generally include three main parts: an external covering layer, an absorbent core layer and a substrate fabric layer.

The external covering layer: it can be attached to the baby's body, facilitating the quick penetration of the urine and effectively preventing the back-infiltrating thereof, thereby keeping the surface layer of the diaper dry. The diaper in Chinese market is basically of non-woven fabric surface layer. This permeable non-woven fabric can improve the interior permeability of the diaper to render water vapor circulate out of the diaper, enabling to timely discharge moisture and hot air and to effectively reduce the risk of diaper rash, and its softness and comfort will not irritate the skin.

The absorbent core layer: it can capture and absorb the urine quickly and disperse or spread the urine throughout the core layer via capillary action to complete the absorption and storage of the urine. The absorbent core layer of the diaper in the current market is a layered structure mainly composed of pure pulp (fluff pulp) and super absorbent resin (SAP).

The substrate fabric layer: substrate fabrics of a lot of foreign diapers have been made of the nonwoven fabric which is more comfortable. However, most diapers in the market are still mainly composed of PE film due to its lower cost. This kind of diaper usually connects and secures the left and right fore-attachments by use of a hook&loop in form of hook or flannel cloth and of glossy and composite adhesive plastic films. This hook&loop fore-attachments need adhesive, which is not environment-friendly but high cost. While, the cheaper diapers are made of pressure point nonwoven fabric. The disadvantage of this pressure point nonwoven fabric is heavy and thick, and is also of rigid texture which will cause discomfort for users. Moreover, this diaper is easy to loosen or even to fall off since the magic tape is not tight. By adopting glossy and composite adhesive plastic films, the corners and edges of the adhesive tape's plastic material are liable to hurt the baby's skin. Since the waist tape of the diaper is often needed to be loosed repetitively, the thicker plastic film or composite fiber layer material should be selected in order to assure the usage effect, which both increase the cost and cause discomfort.

### SUMMARY OF THE INVENTION

For overcoming the above drawbacks in the prior art, the purpose of the present application is to provide a fore-attached composite structure for diaper, which has advantages of light, environmentally-friendly, reliable connection and low cost.

According to one aspect of the present invention, it provides a fore-attached composite structure for diaper, which comprises a substrate layer formed by a polymer film composite and a hook&loop. The surface of the substrate layer is composited with a fiber layer of a thermoplastic polymer. The substrate layer formed by a polymer film composite is light, soft, and permeable. The fiber layer made of thermoplastic polymer woven or nonwoven fiber is soft to touch and firmly connected to the hook&loop.

The present invention further comprises one or a combination of the following technical features.

The fiber layer is made of woven or nonwoven fiber composited by the thermoplastic polymer.

Fiber layer is composed of a number of fiber filaments composited by the thermoplastic polymer, preferably continuous synthetic filaments including spinning fibers and multi-strand fibers.

In order to have a better support, a number of projections are positioned on the surface of the substrate layer. The optimal distribution density of these protrusions is 30,000 to 60,000 per square meter. These projections may or may not have same shape.

In order to achieve a better touch and a stronger connection with the hook&loop, the fiber layer composited on the projections on the surface of the substrate layer is configured to be fluffy; and the fiber layer formed at the recess between each pair of projections is configured to be in a dense status. The connection is closer when the hook&loop contacts the fiber layer at the recess. The fiber layer on the projection makes a soft and comfortable hand-feel.

In order to enrich the appearance of the product to meet the preferences of infants, the substrate layer can be configured to be a transparent layer printed with various cartoon patterns..

The projections distributed on the surface of the substrate layer can be configured to be a geometry structure including structure of square, rectangular, circular, oval, triangular, diamond, etc. The projections distributed on the surface of the substrate layer are arranged regularly or irregularly. When arranged regularly, the projections are in a triangle arrangement in a longitudinal direction, in a linear arrangement in a transverse direction, and are diagonally arranged at 45 degree angle in the right and left sides; the projection has a length from 2mm to 5mm, a width from 2mm to 5mm and a height from 1mm to 3mm. The substrate layer is preferably a transparent or non-transparent layer printed with patterns.

The fore-attached composite structure for diaper of the present invention has the characters of light, environmentally-friendly, comfortable, firm connection to the hook&loop and low cost compared to the conventional fore-attachment, since its double-layer material structure constituted of the continuous filament fiber layer and a fused polymer film substrate layer does not need a glue to achieve bonding.

### BRIEF DESCRIPTION OF THE DRAWINGS

Figure 1 is a planar structure diagram for the square projections according to one embodiment of the present invention;
Figure 2 is a structure schematic diagram for a transverse cross-section of the projections in Figure 1;
Figure 3 is a structure schematic diagram for a longitudinal cross-section of the projections in Figure 1;
Figure 4 is a perspective structure schematic diagram of the projections in Figure 1;
Figure 5 is a planar structure diagram for the diamond projections according to present invention;
Figure 6 is a perspective structure schematic diagram of the projections in Figure 5;
Figure 7 is a structure diagram for the elongated projections according to present invention;
Figure 8 is a structure diagram for the rectangle projections according to present invention;
Figure 9 is a planar structure diagram for the projections in a triangle arrangement according to present invention; and
Figure 10 is a perspective structure schematic diagram of the projections in Figure 9.

### DETAILED DESCRIPTION

For making the purposes, technical solutions, and advantages of the present invention more clear, the present invention will be further described in details in conjunction with the accompanying drawings as follows.

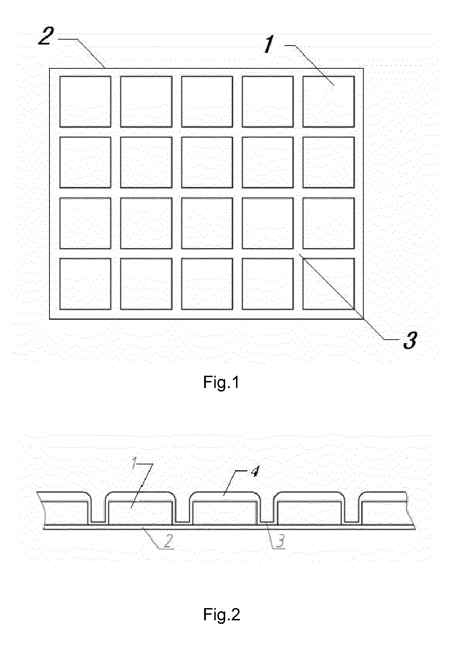

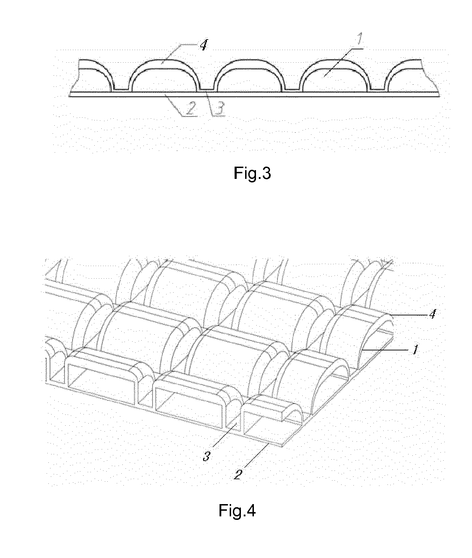

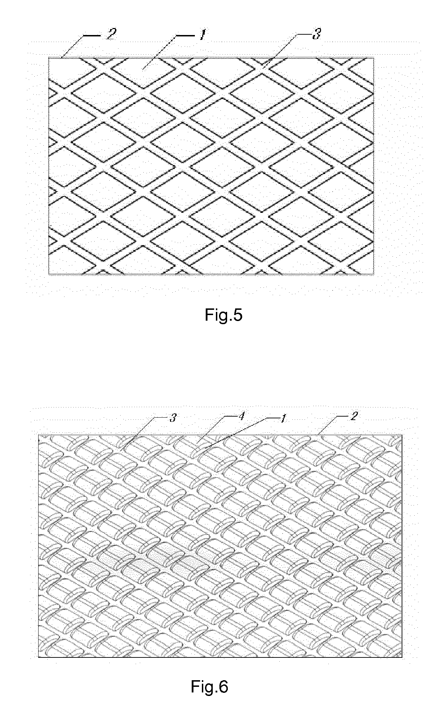

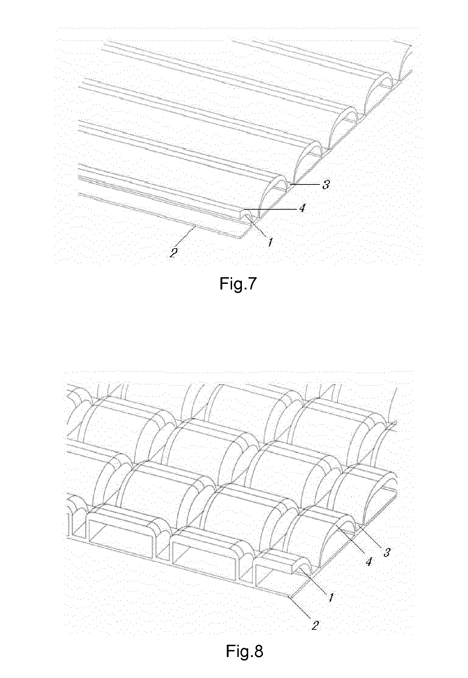

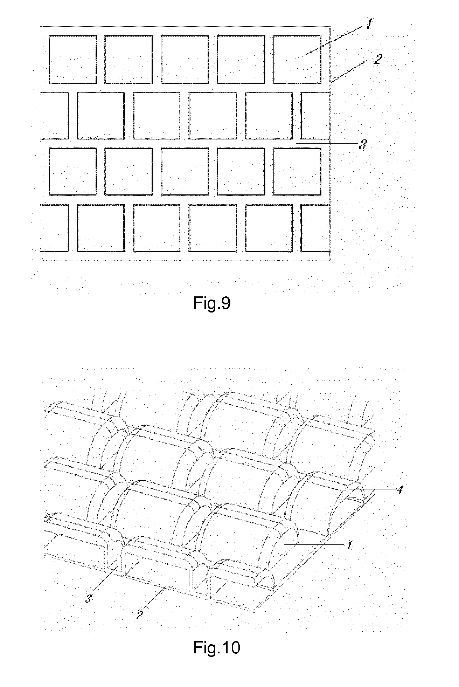

With reference to figures 1 to 10, a fore-attached composite structure for diaper comprises a substrate layer 2 composited by a polymer film, a fiber layer 4 of a thermoplastic polymer woven or nonwoven fiber composited on the surface of the substrate layer 2, and a hook&loop (not shown in the figures) adhered to the fiber layer 4. In order to have a better support, a number of projections 1 are positioned on the surface of the substrate layer 2. The distribution density of these protrusions 1 may be 30,000 to 60,000 per square meter. These projections 1 may or may not have same shape. In order to achieve a better touch and a stronger connection to the hook&loop, the fiber layer 4 disposed on the projections 1 on the surface of the substrate layer 2 is configured to be fluffy; and the fiber layer 4 formed at the recess 3 between each pair of projections 1 is configured to be in a dense status. The connection is closer when the hook&loop contacts the fiber layer 4 at the recess 3. The fiber layers 4 on the projections 1 make the hand feel soft and comfortable. The substrate layer 2 composited by a polymer film is light, soft, and permeable. The fiber layer 4 made of thermoplastic polymer woven or nonwoven fiber is soft to touch and firmly connected to the hook&loop. The fiber layer 4 is composed of a number of fiber filaments composited with the thermoplastic polymer, preferably continuous synthetic filaments including spinning fibers and multi-strand fibers.

In order to enrich the appearance of the product to meet the preferences of infants, the substrate layer 2 may be configured to be a transparent layer printed with various cartoon patterns. The projections 1 distributed on the surface of the substrate layer 2 may be configured to be a geometry structure including a structure of square, rectangular, circular, oval, triangular, diamond, etc. The projections 1 distributed on the surface of the substrate layer 2 are arranged regularly or irregularly. In some regular examples, the projections are preferably in a triangle arrangement in a longitudinal direction, in a linear arrangement in a transverse direction, and are diagonally arranged at 45 degree angle in the right and left sides; the projection has a length from 2mm to 5mm, a width from 2mm to 5mm and a height from hum to 3mm. The substrate layer 2 is a transparent or non-transparent layer printed with patterns.

Compared to the conventional fore-attachments, the fore-attached composite structure for diaper of the present invention has the characters of light, environmentally-friendly, comfortable, firm connection to the hook&loop and low cost since its double-layer material structure constituted of the continuous filament fiber layer and a fused polymer film substrate layer does not need a glue to achieve bonding.

The embodiments described above are only used for illustrating the technical concepts and features of the present invention, aim to make one skilled in the art understand the contents of the present invention and implement accordingly, and cannot be understood as a limit to the protection scopes of the present invention. The equivalent variations and modifications made according to the essential contents and core technical solutions of the present invention shall fall into the protection scopes of the present invention.

## Claims

1. A fore-attached composite structure for diaper, comprising a substrate layer composited by a polymer film and a fiber layer of a thermoplastic polymer composited on the surface of the substrate layer.

2. The fore-attached composite structure for diaper of claim 1, **characterized in that** the surface of the substrate layer has a number of projections.

3. The fore-attached composite structure for diaper of claim 1 or 2, **characterized in that** the fiber layer consists of woven or nonwoven fibers composited with thermoplastic polymers.

4. The fore-attached composite structure for diaper of claim 3, **characterized in that** the projections may or may not have a same shape.

5. The fore-attached composite structure for diaper of claim 2, **characterized in that** the fiber layer composited on the projections is fluffy; and the fiber layer formed at the recess between each pair of projections is in a dense status.

6. The fore-attached composite structure for diaper of claim 1, **characterized in that** the substrate layer is a transparent layer.

7. The fore-attached composite structure for diaper of claim 1 or 6, **characterized in that** the substrate layer is printed with patterns..

8. The fore-attached composite structure for diaper of claim 4, **characterized in that** the projections distributed on the surface of the substrate layer present a geometry structure.

9. The fore-attached composite structure for diaper of claim 8, **characterized in that** the projections distributed on the surface of the substrate layer are arranged regularly or irregularly.

10. The fore-attached composite structure for diaper of claim 8, **characterized in that** the projections are in a triangle arrangement in a longitudinal direction, in a linear arrangement in a transverse direction, and are diagonally arranged at 45 degree angle in the right and left sides;
the projection has a length from 2mm to 5mm, a width from 2mm to 5mm and a height from 1mm to 3mm; and
the substrate layer is transparent or non-transparent layer printed with patterns.
